# EUROPEAN PATENT SPECIFICATION

(11) **EP 1 129 735 B1**
(45) Date of publication and mention of the grant of the patent: **23.08.2006**
(21) Application number: 01301892.4
(22) Date of filing: 01.03.2001
(51) Int. Cl.: A61M 1/00

(54) **Catheter**
Katheter
Cathéter

(30) Priority: 01.03.2000 GB 0005009
(43) Date of publication of application: 05.09.2001
(73) Proprietor: Meddis Limited, Wallingford, Oxfordshire OX10 9DG (GB)
(72) Inventor: Gough, Nicholas James, Pangbourne , Berkshire RG8 7QL (GB)
(74) Representative: Jones, David Colin

(56) References cited:
- EP-A- 0 457 220
- EP-A- 0 586 056
- GB-A- 1 234 415
- US-A- 3 885 565
- US-A- 3 890 976
- US-A- 4 205 677
- US-A- 5 429 596
- US-A- 5 562 077

## Description

This invention relates to a catheter.

In particular the invention relates to a catheter suitable for removing fluid from a patient, comprising a tubing having a distal end for insertion into the patient and a proximal end secured to a vacuum control for connection to a vacuum source for effecting the removal of the fluid. Although the catheter is primarily arranged to remove fluid from a patient, and is provided with a closable vent to assist in this, it will be appreciated that the construction of the catheter also allows its use for the introduction of fluid into the patient. For example, a saline solution or sterile water may be introduced into the patient to dilute secretions that are to be suctioned out through the catheter. The term "fluid" is herein intended to encompass not only gases and liquids of relatively low viscosity, but also fluids carrying particulate matter, and fluids whose viscosity is low enough to allow them to be transported through the catheter given the size of the bore of its tubing.

Such catheters are known for use in endotracheal suctioning of patients, for example patients who are hospitalised, and particularly patients in intensive care units, requiring respiratory suction. Patients who are intubated and ventilated have to breathe with the help of a machine, and commonly their own respiratory system is unable automatically to remove the protective secretions which entrap bacteria, dust particles, airborne pollen etc. For this reason, these patients have to be helped by nursing or physiotherapy staff and suctioning is carried out using a suction catheter connected to a vacuum source. Normally, the suction catheter consists of a PVC tube of suitable length and gauge, and a manually operated vacuum control, which operates the application of the vacuum through the tubing. It is helpful for the operator and quite an important safety feature that the patient's secretions can be observed as they pass through the vacuum control. For this reason, catheters are sometimes provided with a clear plastic vacuum control. Also important, is the rapid identification of the catheter for gauge size at any time, and catheters may be colour coded for this purpose. Two-part vacuum controls are available that allow both characteristics to be incorporated into one component.

In these known catheters, such as that available from Meddis Ltd under the trade mark Caretip for example, a body part of the vacuum control is transparent, and colour coding for tubing gauge (outer diameter) is provided in the form of a collar, or insert, between the body and the catheter tubing. The vacuum control has a first passageway therethrough interconnecting the catheter tubing with a tube to the vacuum source. The vacuum control has a second passageway that intercepts the first passageway and that terminates in an external vent. In use, the operator holds the vacuum control in the palm of the hand so as to allow a finger or thumb to engage the vent to control the degree of suction applied to the patient. However, having selected the appropriate size catheter in accordance with the colour coding of the known catheters, the operator has to open the palm of the hand to view the material removed from the patient as it passes through the main body part of the vacuum control. This action may result in, or necessitate, disadvantageous interruption of the suctioning of the patient.

US 3 885 565 shows a catheter and a suction drainage control connector for use therewith.

It is one object of the present invention to provide a catheter that allows problems or difficulties of known configurations to be overcome or at least alleviated.

Thus, in accordance with one aspect of the present invention, there is provided a catheter for removing fluid from a patient according to claim 1.

For convenience, the second, transparent portion of the vacuum control of the catheter is preferably located adjacent the vacuum vent of the first portion.

Typically, the tubing used in such catheters is frosted, so that its contents cannot be clearly seen, and even when the tubing is of clear material , usually plastic tubing such as PVC, its gauge may be too small to allow an operator properly to observe the material being drawn therethrough. The second portion of the vacuum control, which provides the observation chamber, is preferably chosen to have a volume, and in particular a diameter, that is sufficiently large to allow the operator to identify the material that is being passed therethrough. Depending on the gauge of the catheter tubing actually in use, the size, for example bore, of the observation chamber may be larger than the bore of the catheter tubing.

The first portion of the vacuum control, which is substantially opaque and which is colour coded preferably to the catheter gauge, may also be frosted.

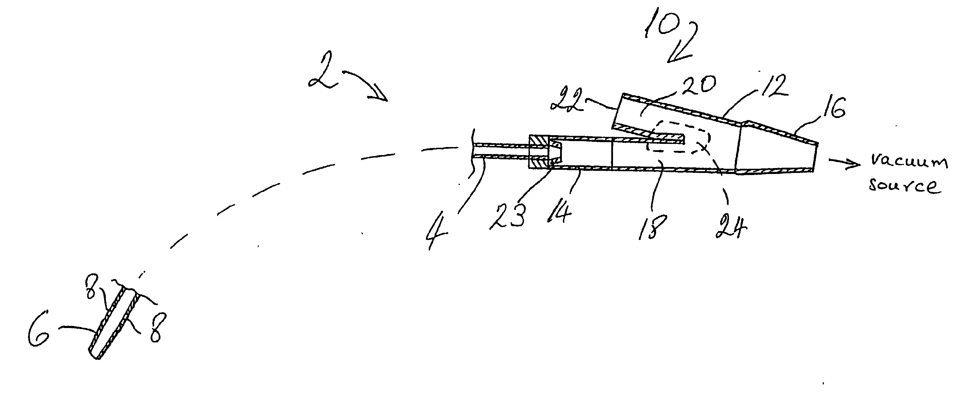

A catheter in accordance with present invention, will now be described, by way of example, with reference to the accompanying drawing, which shows a longitudinal cross section through the catheter.

Referring to the drawing, a catheter 2 comprises a frosted PVC catheter tubing 4 of approximately 50cm length and bore diameter 2mm. The tubing 4 has a frusto-conical distal end 6 which is open-ended, and which has a pair of diametrically - opposed apertures 8 in the side wall. At its other, proximal end, the tubing 4 is secured to a vacuum control 10 of plastics material. The vacuum control 10 comprises a first part 12 which forms a colour-coded opaque plastics main body, and a tubular transparent portion 14 integral therewith and which sealingly receives the end of the tubing 4. The tubing 4 may be retained in the tubular housing 14 by means of adhesive or by being ultrasonically welded thereto. The main body 12 is tapered at its free end 16 for insertion into a further tubing (not shown), for connection to a vacuum source (not shown). A fluid passageway thus exists from the distal end 6 of the catheter 2 along the tubing 4, through the transparent tubing housing 14, and thence along a first passageway 18 of the main body 12 to the vacuum source. The main body 12 also has a second passageway 20 that intersects the first passageway 18 and terminates in a vent 22.

The transparent housing, or observation chamber 14 is provided with an internal conical baffle 23 at its end where it receives the incoming catheter tubing 4. The aperture of the baffle is no smaller than that of the inner lumen of the catheter tubing 4, so that it does not interfere with the free flow of air being sucked out by the vacuum source. The annular region surrounding the baffle 23 within the housing 14 serves securely to trap a specimen of the fluid material removed from the patient, for observation and for later inspection and analysis.

In operation, the tubing 4 is inserted into the respiratory tract of the patient to dispose the distal end 6 in the region of the patient's body from which secretions are required to be removed. The main body 12 is gripped in the palm of a hand, advantageously by means of pads 24 (only one of which is shown) on either side of the main body 12, with the fingers extended towards, but not enclosing, the housing 14. With the vent 22 occluded by a finger of the operator, activation of the vacuum source can then be effective to drawn the secretions from the patient up through the tubing 4 and out through the vacuum control 10. Removal of the finger from the vent 22 allows the vacuum to escape, and thus the suctioning to stop, without removal of the catheter 2 from the patient. It will thus be appreciated, that partial occlusion of the vent 22 can control the degree of suctioning that is applied through the catheter 2.

The provision of the transparent tubular housing 14 between the vacuum control 10 and the catheter tubing 4 allows the operator to observe the material drawn from the patient as it passes through the observation chamber that is formed by the casing 14, and these observations can be conveniently carried out whilst control of the suction is effected by full or partial occlusion of the vent 22 whilst the main body 12 of the vacuum control 10 is retained in the palm of the operator.

Since suctioning, e.g. endotracheal suctioning, may require the tubing of the catheter to be a different gauge for different procedures and/or for different patients, it is important that the gauge, i.e. outer diameter, of the catheter be clearly identified, and that this identification can be carried out even when the tubing 4 is completely disposed within the patient. To this end, the main body 12 of the vacuum control 10 of the catheter 2 is formed of a material having a colour that is coded to the gauge of the tubing 4.

In accordance with the present invention, therefore, there is provided a catheter that has a totally transparent part, which is specifically designed as an observation port that is always visible during usage and which is situated just in front of the fingers holding the vacuum control. The operator thus has the ability to recognise any signs of infection or tissue trauma whilst working with the patient, at the same time as working efficiently and quickly to reduce trauma and discomfort. The catheter is also provided with a further part that is colour coded to indicate the gauge of the catheter.

## Claims

1. A catheter (2) for removing fluid from a patient, comprising a tubing (4) having a distal end (6) for insertion into the patient and a proximal end secured to a vacuum control (10) for connection to a vacuum source for effecting the removal of the fluid, wherein the vacuum control (10) comprises a first portion (12) arranged to be gripped by an operator of the catheter whereby the degree of suction applied to the catheter tubing (4) from the vacuum source may be manually controlled by occlusion of a vent (24) of the first portion (12), and a second portion (14) that extends from the first portion (12) to receive the catheter tubing (4) therein, **characterised in that** the first portion (12) of the vacuum control (10) is substantially opaque and is colour coded so as to be indicative of the diameter of the catheter tubing (4) secured to the vacuum control (10), and **in that** the second portion (14) is substantially transparent and defines an observation chamber between the first portion (12) of the vacuum control (10) and the catheter tubing (4).

2. A catheter (2) according to claim 1, wherein the second, transparent portion (14) of the vacuum control (10) is located adjacent the vacuum vent (24) of the first portion (12) thereof.

3. A catheter (2) according to claim 1 or claim 2, wherein the observation chamber of the second portion (14) is tubular and has a bore that is larger than the bore of the catheter tubing (4).

4. A catheter (2) according to any one of the preceding claims, wherein the catheter tubing (4) is opaque, or only partially transparent.

5. A catheter (2) according to claim 4, wherein the catheter tubing (4) is frosted.

## Patentansprüche

1. Katheter (2) zur Flüssigkeitsausleitung aus einem Patienten, umfassend einen Schlauch (4) mit einem distalen Ende (6) zum Einsetzen in den Patienten und einem proximalen Ende, das an einer Saugluftsteuerung (10) zum Anschluss an eine Vakuumquelle befestigt ist, um die Flüssigkeitsausleitung zu bewirken, wobei die Saugluftsteuerung (10) einen ersten Abschnitt (12) aufweist, der von der den Katheter legenden Person ergriffen werden soll, wodurch das von der Vakuumquelle an den Katheterschlauch (4) gelegte Maß an Sog durch Verschließen einer Abzugsöffnung (22) des ersten Abschnitts (12) manuell geregelt werden kann, sowie einen vom ersten Abschnitt (12) weg verlaufenden zweiten Abschnitt (14) zur Aufnahme des Katheterschlauchs (4) darin, **dadurch gekennzeichnet, dass** der erste Abschnitt (12) der Saugluftsteuerung (10) im Wesentlichen undurchsichtig ist sowie zur Angabe des Durchmessers des an der Saugluftsteuerung (10) angebrachten Katheterschlauchs (4) farblich **gekennzeichnet** ist, und dass der zweite Abschnitt (14) im Wesentlichen transparent ist und zwischen dem ersten Abschnitt (12) der Saugluftsteuerung (10) und dem Katheterschlauch (4) eine Beobachtungs- oder Einblickkammer definiert.

2. Katheter (2) nach Anspruch 1, **dadurch gekennzeichnet, dass** der zweite transparente Abschnitt (14) der Saugluftsteuerung (10) in der Nähe der Vakuumabzugsöffnung (22) ihres ersten Abschnitts (12) angeordnet ist.

3. Katheter (2) nach Anspruch 1 oder Anspruch 2, **dadurch gekennzeichnet, dass** die Beobachtungskammer des zweiten Abschnitts (14) röhrenförmig ist und ihre Bohrung größer als die Bohrung des Katheterschlauchs (4) ist.

4. Katheter (2) nach einem der vorangehenden Ansprüche, **dadurch gekennzeichnet, dass** der Katheterschlauch (4) undurchsichtig oder nur teilweise transparent ist.

5. Katheter (2) nach Anspruch 4, **dadurch gekennzeichnet, dass** der Katheterschlauch (4) mattiert ist.

## Revendications

1. Cathéter (2) destiné à l'extraction d'un fluide d'un patient, comprenant un tube (4) ayant une extrémité distale (6) destinée à l'insertion dans le patient et une extrémité proximale fixée à un organe (10) de réglage de vide destiné à être raccordée à une source de vide pour l'exécution de l'extraction du fluide, dans lequel l'organe (10) de réglage de vide comprend une première portion (12) destinée à être saisie par un opérateur du cathéter, de manière que le degré d'aspiration appliqué au tube (4) du cathéter par la source de vide puisse être réglé manuellement par occlusion d'un organe de ventilation (24) de la première portion (12), et une seconde portion (14) qui s'étend depuis la première portion (12) afin qu'elle loge le tube (4) du cathéter, **caractérisé en ce que** la première portion (12) de l'organe (10) de réglage de vide est pratiquement opaque et de couleur codée pour être représentative du diamètre du tube (4) du cathéter fixé à l'organe (10) de réglage de vide, et **en ce que** la seconde portion (14) est pratiquement transparente et délimite une chambre d'observation entre la première portion (12) de l'organe (10) de réglage de vide et le tube (4) du cathéter.

2. Cathéter (2) selon la revendication 1, dans lequel la seconde portion transparente (14) de l'organe (10) de réglage de vide est adjacente à l'organe (24) de ventilation du vide de sa première portion (12).

3. Cathéter (2) selon la revendication 1 ou 2, dans lequel la chambre d'observation de la seconde portion (14) est tubulaire et a un trou plus grand que le trou du tube (4) du cathéter.

4. Cathéter (2) selon l'une quelconque des revendications précédentes, dans lequel le tube (4) du cathéter est opaque ou seulement partiellement transparent.

5. Cathéter (2) selon la revendication 4, dans lequel le tube (4) du cathéter est dépoli.
